# EUROPÄISCHE PATENTANMELDUNG

(11) **EP 0 657 470 A2**
(43) Veröffentlichungstag der Anmeldung: **14.06.1995**
(21) Anmeldenummer: 94103647.7
(22) Anmeldetag: 10.03.1994
(51) Int. Cl.: C07K 16/18, C12N 5/20, C12P 21/08, C07K 14/765, C07K 1/22, G01N 33/68, G01N 33/577

(54) **Monoklonale Antikörper gegen in vivo glykiertes Albumin**

(30) Priorität: 03.12.1993 DE 4308532
(71) Anmelder: BOEHRINGER MANNHEIM GMBH, D-68298 Mannheim (DE)
(72) Erfinder: Schlipfenbacher, Reiner, D-67098 Bad Dürkheim (DE); Hallermayer, Klaus, D-80339 München (DE); Essig, Ulrich, D-82152 Planegg (DE); Huebner-Parajsz, Christa, D-82327 Tutzing (DE)

(57) **Zusammenfassung**

Gegenstand der Erfindung ist ein monoklonaler Antikörper gegen in vivo glykiertes Albumin, welcher zu weniger als 30 % mit nicht glykiertem Albumin reagiert, ein Verfahren zur Herstellung eines solchen monoklonalen Antikörpers, sowie die Verwendung eines solchen monoklonalen Antikörpers zum spezifischen Nachweis von in vivo glykiertem Albumin.

## Beschreibung

Gegenstand der Erfindung ist ein monoklonaler Antikörper gegen in vivo glykiertes Albumin, welcher zu weniger als 30 % mit nicht glykiertem Albumin reagiert, ein Verfahren zur Herstellung eines solchen monoklonalen Antikörpers, sowie die Verwendung eines solchen monoklonalen Antikörpers zum spezifischen Nachweis von in vivo glykiertem Albumin.

Albumin ist ein Serumprotein mit einem Molekulargewicht von 66 kD und besteht aus 585 Aminosäuren. Es enthält 60 Lysinreste, an denen das Albumin glykiert werden kann. Albumin wird daher, wie auch andere im Blutstrom zirkulierende Plasmaproteine, nichtenzymatisch glykiert. Diese nichtenzymatische Glykierung ist eine langsam, kontinuierlich und irreversibel ablaufende Reaktion, die im wesentlichen von der Blutglukosekonzentration abhängig ist. Das Ausmaß der Glykierung von Proteinen aus dem Blut ist daher ein wichtiger Indikator für die Bestimmung des mittleren Blutglukosespiegels. Die Bestimmung von glykiertem Hämoglobin erfaßt dabei wegen der langen Halbwertszeit von Hämoglobin nur langfristige Änderungen des Blutglukosespiegels. Albumin weist eine wesentlich kürzere biologische Halbwertzeit von etwa 19 Tagen auf, so daß glykiertes Albumin einen wichtigen Parameter zur Kontrolle von mittelfristigen Veränderungen des Blutglukosespiegels darstellt (E. Hud et al., Clin. Chem. Acta 185 (1989), 157-164).

Glykiertes Albumin kann nach Shima et al. (K. Shima et al., Diabetologica 31 (1988), 627 - 631 und K. Jasukawa et al., J. Chromatography 597 (1992), 271) chromatographisch isoliert und nachgewiesen werden. Dieses Verfahren ist jedoch für einen diagnostischen Test zu aufwendig. Es wurden daher Antikörper gegen glykiertes Albumin entwickelt. Diese Antikörper zeigen jedoch für einen diagnostischen Test eine zu hohe Kreuzreaktivität mit anderen (glykierten oder nicht glykierten) Serumproteinen oder mit nicht glykiertem Albumin. In der EP-A 0 257 421 werden Antikörper beschrieben, die spezifisch für die Glykierung an Lysin 525 des Albumins sind. Diese Antikörper erkennen somit nur ein spezielles glykiertes Albumin. Von M. Cohen et al. wurde ein weiterer monoklonaler Antikörper (A717) gegen glykiertes Albumin entwickelt (J. of Immunol. Meth. 117 (1989), 121-129). Dieser Antikörper differenziert zwar zwischen in vitro glykiertem Albumin und nicht glykiertem Albumin, nicht jedoch zwischen in vivo glykiertem Albumin und nicht glykiertem Albumin. Dieser Unterschied ist damit zu erklären, daß in vitro glykiertes Albumin stärker glykiert ist als in vivo glykiertes Albumin und sich daher auch stärker von nicht glykiertem Albumin unterscheidet. Auch die in WO 88/00346 beschriebenen monoklonalen Antikörper gegen glykierte Plasmaproteine zeigen lediglich eine Differenzierung zwischen in vitro glykiertem und nicht glykiertem Plasmaprotein. Selbst dabei weist der beste Antikörper (74-G11) noch eine Reaktivität von etwa 40 % (bezogen auf die Reaktivität mit in vitro glykierten Plasmaproteinen) mit nicht glykierten Plasmaproteinen auf.

Aufgabe der Erfindung war es daher, einen monoklonalen Antikörper gegen glykiertes Albumin zur Verfügung zu stellen, der eine Differenzierung zwischen in vivo glykiertem Albumin und nicht glykiertem Albumin ermöglicht.

Diese Aufgabe wird gelöst durch einen monoklonalen Antikörper gegen in vivo glykiertes Albumin, der zu weniger als 30 % (bezogen auf die Reaktivität mit in vivo glykiertem Albumin) mit nicht glykiertem Albumin reagiert, und erhältlich ist durch Immunisierung mit in vivo glykiertem Albumin, das aus Serum über Immunaffinitätschromatographie und Boronatchromatographie aufgereinigt wurde, Immortalisierung der Milzzellen der immunisierten Tiere und Klonierung derjenigen immortalisierten Zellen, deren Kulturüberstand den gewünschten Antikörper gegen in vivo glykiertes Albumin enthält.

Es hat sich überraschenderweise gezeigt, daß die erfindungsgemäßen monoklonalen Antikörper auch dann nur eine geringe Reaktivität (unter 30 %) mit nicht glykiertem Albumin aufweisen, wenn diese Reaktivität auf die Reaktivität mit in vivo glykiertem Albumin bezogen wird, welches weniger stark glykiert ist als in vitro glykiertes Albumin.

Das in vivo glykierte Albumin wird aus Serum isoliert. Vorzugsweise wird ein Diabetikerserum mit erhöhtem Fructosamingehalt, das heißt mit 400 - 500 µmol/l Fructosamin verwendet. Der Fructosamingehalt wird in üblicher Weise, z.B. mit der Test-Combination Fructosamin (Boehringer Mannheim GmbH, Katalog Nr. 1298194) bestimmt. Zur Aufreinigung wird aus diesem Serum zunächst die Immunglobulinfraktion mit Ammoniumsulfat ausgefällt und der Überstand gegen Natriumchlorid-haltigen Phosphatpuffer dialysiert. Anschließend wird Albumin über eine Affinitätschromatographie an einer polyklonalen Immunglobulin-G-Fraktion gegen Humanserumalbumin isoliert. Die erhaltene Gesamtalbuminfraktion wird gegen Ammoniumacetatpuffer dialysiert und das glykierte Albumin über eine Chromatographie an Boronat abgetrennt. Dabei können alle geläufigen Trägermaterialien für die Boronatgruppen verwendet werden, vorzugsweise wird als Trägermaterial für die Boronatchromatographie Fractogel TSK-AF Phenylboronat verwendet. Als Äquilibrierungs- und Waschpuffer wird vorzugsweise ethanolhaltiges Ammoniumacetat verwendet. Die Elution erfolgt vorzugsweise mit Tris-Puffer, der Sorbit enthält. Das so isolierte, glykierte Albumin wird gegen Phosphatpuffer dialysiert und über HIC (Hydrophobic Interaction Chromatography) weiter aufgereinigt.

Mit dem auf diese Weise isolierten in vivo glykierten Albumin als Immunogen erfolgt die Immunisierung der hierfür üblicherweise verwendeten Tiere. Vorzugsweise werden Mäuse verwendet.

Die Immortalisierung der Milzzellen der immunisierten Tiere erfolgt nach bekannten Verfahren, zum Beispiel durch die Fusion mit Myelomzellen wie z.B. der Myelomzellinie P3X63-Ag8-653 (ATCC CRL 1580). Vorzugsweise erfolgt die Fusion gemäß dem von Galfré, Methods in Enzymology 173, 1981, 3, beschriebenen Verfahren.

Der Kulturüberstand der immortalisierten Zellen wird nach üblichen Verfahren, zum Beispiel über einen ELISA-Test, auf Reaktivität mit in vivo glykiertem und nicht glykiertem Albumin getestet. Diejenigen Zellen, deren Kulturüberstand hierbei eine positive Reaktion mit in vivo glykiertem Albumin, aber keine oder nur eine geringe Reaktion mit nicht glykiertem Albumin ergibt, werden in üblicher Weise, zum Beispiel über einen fluoreszenzaktivierten Zellsorter, vereinzelt und kloniert.

Ein bevorzugter Gegenstand der Erfindung sind monoklonale Antikörper gegen in vivo glykiertes Albumin, welche nach dem oben beschriebenen Verfahren erhältlich sind und zu weniger als 25 %, besonders bevorzugt weniger als 10 %, ganz besonders bevorzugt zu weniger als 1 % mit nicht glykiertem Albumin reagieren. Besonders bevorzugt sind solche monoklonalen Antikörper, die in äquivalenter Weise an in vivo glykiertes Albumin bindefähig sind, wie die von den Zellinien MAK 2.21.11 oder MAK 2.6.28 produzierten monoklonalen Antikörper sowie insbesondere die aus diesen Zellinien erhältlichen monoklonalen Antikörper.

Unter dem Begriff "in äquivalenter Weise bindefähiger Antikörper" werden Antikörper verstanden, bei denen eine Epitopüberlappung mit dem definierten, bekannten Antikörper nachweisbar ist. Diese Epitopüberlappung kann mit Hilfe eines kompetitiven Testsystems leicht nachgewiesen werden. Dazu wird zum Beispiel mit Hilfe eines Enzym-Immunoassays überprüft, inwieweit ein Antikörper mit dem bekannten Antikörper um die Bindung an ein definiertes Antigen bzw. ein spezielles Epitop konkurriert. Dazu inkubiert man das entsprechende Antigen mit dem bekannten monoklonalen Antikörper in markierter Form und einem Überschuß des in Betracht gezogenen Antikörpers. Durch Immobilisierung der gebildeten Komplexe, Trennung der festen von der flüssigen Phase und Nachweis der gebundenen Markierung in einer der beiden Phasen kann dann leicht festgestellt werden, inwieweit der in Betracht gezogene Antikörper den definierten Antikörper aus der Bindung verdrängen kann. Ist eine Verdrängung von mindestens 50 % bei 10⁵-fachem Überschuß gegeben, so liegt eine Epitopüberlappung vor.

Ein weiterer Gegenstand der Erfindung sind die Zellinien MAK 2.21.11 und MAK 2.6.28.

Ein weiterer Gegenstand der Erfindung ist ein Verfahren zur Herstellung eines erfindungsgemäßen monoklonalen Antikörpers gegen in vivo glykiertes Albumin, welches dadurch gekennzeichnet ist, daß man mit in vivo glykiertem Albumin immunisiert, das aus Serum über Immunaffinitätschromatographie und Boronatchromatographie aufgereinigt wurde, die Milzzellen der immunisierten Tiere immortalisiert und diejenigen immortalisierten Zellen kloniert, deren Kulturüberstand den gewünschten Antikörper gegen glykiertes Albumin enthält.

Es hat sich überraschenderweise gezeigt, daß ein auf die beschriebene Weise isoliertes, in vivo glykiertes Albumin ein besonders geeignetes Immunogen darstellt, um monoklonale Antikörper gegen in vivo glykiertes Albumin zu erhalten, welche eine geringe Kreuzreaktivität mit nicht glykiertem Albumin aufweisen.

Ein weiterer Gegenstand der Erfindung ist daher ein Verfahren zur Aufreinigung von in vivo glykiertem Albumin, welches dadurch gekennzeichnet ist, daß man Albumin aus Serum über Immunaffinitätschromatographie aufreinigt und das in vivo glykierte Albumin über eine Boronatchromatographie abtrennt.

Mit den erfindungsgemäßen monoklonalen Antikörpern ist eine immunologische Differenzierung zwischen in vivo glykiertem Albumin und nicht glykiertem Albumin möglich. Daher eignen sich die erfindungsgemäßen monoklonalen Antikörper für einen diagnostischen Nachweis von in vivo glykiertem Albumin in Serumproben.

Ein weiterer Gegenstand der Erfindung ist daher die Verwendung eines erfindungsgemäßen monoklonalen Antikörpers in einem immunologischen Nachweisverfahren zur spezifischen Bestimmung von in vivo glykiertem Albumin.

Diese Bestimmung kann über alle gängigen Immunoassays wie z.B. ELISA-Fluoreszenzimmunoassay, Radioimmunoassay , Fluoreszenz-Polarisationsimmunoassay, CEDIA oder EMIT erfolgen. Der Test kann dabei sowohl als homogener Test, z.B. über einen kompetitiven Immunoassay, als auch als heterogener Test durchgeführt werden. Vorzugsweise erfolgt der Test unter Immobilisierung einer der Reaktionspartner.

Ein weiterer Gegenstand der Erfindung ist daher die Verwendung eines erfindungsgemäßen monoklonalen Antikörpers zum spezifischen Nachweis von in vivo glykiertem Albumin, welches dadurch gekennzeichnet ist, daß man die zu analysierende Probe mit dem Antikörper inkubiert, die gebildeten Komplexe vor oder nach der Bindungsreaktion immobilisiert und die Bindung des Antikörpers an das Antigen in üblicher Weise bestimmt.

Der Nachweis der Bindung des Antikörpers an das glykierte Albumin der zu analysierenden Probe erfolgt vorzugsweise in einem Sandwich ELISA-Test. Dazu werden Mikrotiterplatten zunächst mit einem polyklonalen Antiserum gegen Serumalbumin beschichtet und dann mit der zu analysierenden Probe inkubiert. Dabei binden sowohl glykiertes als auch nicht glykiertes Albumin an das immobilisierte, polyklonale Antiserum. Anschließend wird mit einem erfindungsgemäßen monoklonalen Antikörper gegen in vivo glykiertes Albumin inkubiert, welcher nur an das im ersten Schritt immobilisierte in vivo glykierte Albumin bindet. Die Bindung des erfindungsgemäßen Antikörpers wird dann über einen markierten Antikörper gegen murines Fcγ derjenigen Tierspezies, aus welcher der erfindungsgemäße Antikörper erhalten wurde, nachgewiesen. Alternativ kann der erfindungsgemäße Antikörper auch direkt markiert werden. Die Markierung erfolgt in üblicher Weise, zum Beispiel über ein Enzym, eine Fluoreszenz- oder Chemilumineszenzmarkierung.

Ein weiterer Gegenstand der Erfindung ist ein immunologisches Verfahren zur spezifischen Bestimmung von in vivo glykiertem Albumin, bei welchem mindestens ein erfindungsgemäßer monoklonaler Antikörper verwendet wird. Dieses immunologische Verfahren kann nach allen oben genannten Immunoassays erfolgen, wie insbesondere Sandwich-Assay, kompetitiver oder homogener Test. Vorzugsweise wird bei einem solchen immunologischen Verfahren die zu analysierende Probe mit dem Antikörper inkubiert, die gebildeten Komplexe vor oder nach der Bindungsreaktion immobilisiert und die Bindung des Antikörpers an das Antigen in üblicher Weise bestimmt.

Die beiden Hybridomzellinien MAK 2.21.11 (DSM ACC 2052) und MAK 2.6.28 (DSM ACC 2053), welche einen erfindungsgemäßen monoklonalen Antikörper gegen in vivo glykiertes Albumin produzieren, wurden am 21.10.1992 bei der Deutschen Sammlung für Zellkulturen und Mikroorganismen GmbH, Mascheroderweg 1b, D-33 Braunschweig hinterlegt.

Die Erfindung wird durch die folgenden Beispiele weiter erläutert.

### Beispiel 1:

### Isolierung von in vivo glykiertem Albumin aus Humanserum

1. Ammoniumsulfatfällung
   Aus einem Diabetikerserum mit erhöhtem Fructosamingehalt (400 - 500 µmol/l) wird zunächst die Immunglobulinfraktion durch Zugabe von Ammoniumsulfat ad 2,85 mol/l und eine Stunde Rühren bei Raumtemperatur ausgefällt. Der Niederschlag wird 20 min bei 12000 g abzentrifugiert und verworfen. Der Albumin-haltige Überstand wird gegen 50 mmol/l Kaliumphosphatpuffer pH 6,8 / 50 mmol/l Natriumchlorid dialysiert. Anschließend wird mit dem gleichen Puffer eine Proteinkonzentration von 10 mg/ml eingestellt.
2. Immunaffinitätschromatographie
   a) Herstellung des Immunadsorbens
      An Glutardialdehyd-aktiviertes Amino-Spherosil (Trägermaterial auf Silikatbasis, Boehringer Mannheim GmbH, Katalog Nr. 665 525) wird gemäß Angaben des Herstellers eine für Humanserumalbumin spezifische polyklonale Immunglobulin-G-Fraktion aus einem Schaf, welche immunsorbtiv gereinigt wurde, gebunden. Das resultierende Adsorbens hat eine Bindekapazität von etwa 3 mg humanem Serumalbumin pro ml Adsorbens.
   b) Immunadsorbtion
      Das Immunadsorbens aus 2a) wird mit 50 mmol/lKaliumphosphatpuffer pH 6,8 / 50 mmol/l Natriumchlorid äquilibriert. Die Albuminfraktion aus 1) wird dann innerhalb von 2 Stunden bei Raumtemperatur aufgetragen. Anschließend wird das nichtgebundene Protein mit 5 Säulenvolumen 250 mmol/l Kaliumphosphatpuffer pH 6,8 ausgewaschen.
      Das gebundene Gesamtalbumin wird mit 2 Säulenvolumen 0,1% HCl bei Raumtemperatur eluiert und sofort gegen 1 mol/l Ammoniumacetat pH 8,5 dialysiert.
3. Boronatchromatographie
   Die Abtrennung des glykierten Albumins aus dem Gesamtalbumin erfolgt durch Chromatographie an Fractogel TSK AF-Phenylboronat (Merck). Das Chromatographiematerial wird mit 1 mol/l Ammoniumacetat pH 8,5, welches 5% (v/v) Ethanol enthält, äquilibriert.
   Die Gesamtalbuminfraktion aus 2b) wird zunächst in einer Amiconzelle (YM10-Membran) auf 10mg Protein pro ml aufkonzentriert, Ethanol ad 5% (v/v) zugegeben und die erhaltene Lösung bei Raumtemperatur innerhalb von 2 Stunden auf die Säule aufgetragen. Anschließend wird das nichtgebundene Protein mit 4 Säulenvolumen Äquilibrierungspuffer innerhalb von 1 Stunde ausgewaschen. Das gebundene glykierte Albumin wird dann mit 1 - 2 Säulenvolumen Elutionspuffer (100 mmol/l Tris/HCl pH 8,5 mit 800 mmol/l Sorbit) bei Raumtemperatur innerhalb von einer Stunde eluiert und unmittelbar anschließend gegen 50 mmol/l Kaliumphosphatpuffer pH 7,9 dialysiert.
4. HIC-Chromatographie
   Die Aufreinigung des isolierten in vivo glykierten Albumins erfolgt mittels HPLC an einer Phenyl-TSK 5PW Säule (150mm x 21,5mm; Tosohaas, Katalog Nr. 07656).
   Das Eluat der Boronatsäule aus 3. wird auf ca. 8 mg Protein pro ml aufkonzentriert, Ammoniumsulfat ad 1 mol/l zugegeben und über ein 0,45 µm Filter filtriert. Von der erhaltenen Lösung werden pro Lauf der HPLC maximal 1 ml injiziert.
   Die HPLC wird mit dem folgenden Puffergradienten durchgeführt:
   0 - 25 min 100% A, 0% B
   25 - 30 min linearer Gradient bis 40% B
   30 - 60 min linearer Gradient bis 100% B
   60 - 130 min 100% B
   130 - 135 min linearer Gradient bis 100% A, 0% B
   Puffer A: 1 mol/l Ammoniumsulfat / 50 mmol/l Kaliumphosphat pH 7,0
   Puffer B: 50 mmol/l Kaliumphosphatpuffer pH 7,0
   Die Auftrennung erfolgt mit einer Flußrate von 5 ml/min bei Raumtemperatur.
   Das Eluat wird fraktioniert aufgefangen und diejenigen Fraktionen gepoolt, die gemäß SDS Polyacrylamidgel-Elektrophorese (PHAST-System Pharmacia) eine Reinheit von über 99% aufweisen. Diese Fraktionen werden dann gegen 15 mmol/l Kaliumphosphatpuffer pH 7,0 / 50 mmol/l Natriumchlorid dialysiert, auf 3 - 5 mg Protein pro ml aufkonzentriert und bei -80°C gelagert.

### Beispiel 2:

### Herstellung von monoklonalen Antikörpern gegen in vivo glykiertes Albumin

1. Immunisierung von Mäusen mit glykiertem Albumin
   12 Wochen alte Balb/c Mäuse werden mit 100 µg glykiertem Albumin (Isolierung gemäß Beispiel 1) in komplettem Freundschem Adjuvans (CFA) intraperitoneal erstimmunisiert. Nach 6 Wochen erfolgen drei weitere Immunisierungen in jeweils 4-wöchigen Abständen. Dabei werden jeweils 100 µg glykiertes Albumin in inkomplettem Freundschem Adjuvans (IFA) intraperitoneal verabreicht. Daran anschließend erfolgen noch 2 weitere Immunisierungen mit 100 µg glykiertem Albumin in PBS intravenös am 4. und 3. Tag vor der Fusion von Milzzellen der immunisierten Mäuse mit Myelomzellen.
2. Immortalisierung und Klonierung
   Die Fusion von Milzzellen der gemäß 1. immunisierten Mäuse mit Myelomzellen erfolgt in Anlehnung an Galfré, Methods in Enzymology 73, 1981, 3. Dabei werden ca. 1_{*}10⁸ Milzzellen der immunisierten Maus mit 2_{*}10⁷ Myelomzellen (P3X63-Ag8-653, ATCC CRL1580) gemischt und abzentrifugiert (10 min bei 300 g und 4°C). Die Zellen werden dann einmal mit RPMI 1640-Medium ohne fötales Kälberserum (FKS) gewaschen und bei 400 g in einem 50 ml Spitzröhrchen erneut abzentrifugiert. Der Überstand wird abgekippt, das Zellsediment durch Klopfen leicht aufgelockert, 1 ml PEG (Molekulargewicht 4000, Merck, Darmstadt) dazugegeben, und durch Pipettieren gemischt. Nach 1 min im Wasserbad bei 37°C werden bei Raumtemperatur 5 ml RPMI 1640 ohne FKS in einem Zeitraum von 4 - 5 min zugetropft. Danach werden 5 ml RPMI 1640 mit 10% FKS in ca. 1 min zugetropft, durchmischt auf 50 ml mit Medium (RPMI 1640 + 10% FKS) aufgefüllt und anschließend 10 min bei 400 g und 4°C zentrifugiert. Die sedimentierten Zellen werden in RPMI 1640 Medium mit 10% FKS aufgenommen und in Hypoxanthin-Azaserin Selektionsmedium (100 mmol/l Hypoxanthin, 1 µg/ml Azaserin in RPMI 1640 mit 10% FKS) eingesät. Als Wachstumsfaktor wird dem Medium Interleukin 6 (Boehringer Mannheim GmbH, Katalog Nr. 1271 172, 100 U/ml) zugegeben.
   Ca. 1 Woche nach der Fusion sind bereits viele Klone sichtbar. Der Überstand der Primärkulturen wird im ELISA getestet (s. Beispiel 3). Primärkulturen, welche Antikörper gegen glykiertes Albumin produzieren, werden über einen fluoreszenzaktivierten Zellsorter in 96-well-Mikrotiterplatten der Firma Nunc durch Einzelzellablage kloniert. Dem Medium für die Klonierung werden wiederum 100 Units Interleukin 6 pro ml zugesetzt.

### Beispiel 3

### Bestimmung der Spezifität der erhaltenen monoklonalen Antikörper

Die Spezifität der erhaltenen Antikörper wird in einem ELISA-Test bestimmt. Dazu werden zunächst je Vertiefung einer Mikrotiterplatte 100 µl einer Lösung eines immunsorbtiv gereinigten polyklonalen Antiserums aus dem Schaf gegen Humanserumalbumin (10 µg/ml in Beschichtungspuffer = 0,2 mol/l Natriumcarbonat/Bicarbonat pH 9,3 - 9,5) für 1 Stunde bei Raumtemperatur unter Schütteln inkubiert und anschließend 3 mal mit 0.9% Natriumchlorid/0.05% Tween 20 gewaschen. Unspezifische Bindungsstellen werden dann durch eine Nachbeschichtung mit 200 µl 0.5% Crotein C (in PBS) je Vertiefung für 30 min bei Raumtemperatur blockiert und anschließend nochmals 3 mal gewaschen.

In die so vorbereiteten Mikrotiterplatten werden je Vertiefung 100 µl einer Lösung von glykiertem Albumin in PBS (1 µg/ml, Isolierung gemäß Beispiel 1) gegeben und 1 Stunde bei Raumtemperatur unter Schütteln inkubiert.

Kontrollansätze werden parallel mit 100 µl einer Lösung von nicht glykiertem Albumin in gleicher Weise behandelt. Nicht glykiertes Albumin wird erhalten aus Humanserumalbumin (Behring-Werke, Katalog Nr. ORHA 20/21) durch oxidative Abspaltung der Zuckerreste mit Natriumperjodat (2.5 mmol/l in 30 mmol/l Natriumacetatpuffer pH 5.5 bei 0°C für 45 Minuten), Entfernen von überschüssigem Perjodat über Sephadex G-25 und Reaktion der bei der Oxidation erhaltenen reaktiven Aldehydgruppen mit Semicarbazid (3 mmol/l in 30 mmol/l Natriumacetat/Natriumcarbonat pH 7.5, Inkubation 1 Stunde bei 25°C und Dialyse gegen 15 mmol/l Kaliumphosphat/50 mmol/l Natriumchlorid pH 7.5).

Nach 3maligem Waschen mit 0.9% Natriumchlorid/0.05% Tween 20 werden je Vertiefung 100 µl des Zellkulturüberstands von Beispiel 2 zugegeben und 1 Stunde bei Raumtemperatur unter Schütteln inkubiert. Anschließend wird erneut 3 mal gewaschen und dann gebundener Antikörper durch Zugabe von 100 µl je Vertiefung eines Peroxidase-markierten polyklonalen Antiserums aus dem Schaf gegen FCγ der Maus (immunsorbtiv gereinigt, 20 mU/ml) nachgewiesen. Hierzu wird 1 Stunde bei Raumtemperatur unter Schütteln inkubiert, anschließend 3 mal gewaschen und die Nachweisreaktion durch Zugabe von 100 µl je Vertiefung ABTS® ausgelöst. Nach 30 min bei Raumtemperatur erfolgt die Messung bei 405/490 nm im Dynatech-ELISA-Reader MR700.

Die Ergebnisse sind in der folgenden Tabelle zusammengefaßt. Der bei Inkubation ohne den Analyten Albumin erhaltene Leerwert ist jeweils in Klammern angegeben.

| | Reaktivität mit in vivo glykiertem Albumin | | Reaktivität mit nicht glykiertem Albumin | |
|---|---|---|---|---|
| | E nach 30 min | | E nach 30 min | |
| | 5µg AK/ml | 1µg AK/ml | 5µg AK/ml | 1µg AK/ml |
| MAK 2.21.11 | 1,091 | 0,980 | 0,255 | 0,166 |
| (Leerwert) | (0,260) | (0,165) | (0,260) | (0,165) |
| MAK 2.42.29 | 0.661 | 0,596 | 0,109 | 0,065 |
| (Leerwert) | (0,110) | (0,065) | (0,110) | (0,065) |
| MAK 2.6.28 | 0,664 | 0,534 | 0,085 | 0,044 |
| (Leerwert) | (0,090) | (0,045) | (0,090) | (0,045) |
| MAK A717*⁾ | 0,072 | 0,027 | 0,087 | 0,030 |
| (Leerwert) | (0,070) | (0,025) | (0,070) | (0,025) |

| | | | | |
|---|---|---|---|---|
| *⁾ M. Cohen et al., J. of Immunol. Meth. 117 (1989), 121 - 129 | | | | |

### Beispiel 4

### Bestimmung der Menge an glykiertem Albumin in einer Probe

Die Menge an glykiertem Albumin in einer Probe wird in einem ELISA-Test analog Beispiel 3 bestimmt.

In die so wie in Beispiel 3 beschrieben vorbereiteten Mikrotiterplatten werden je Vertiefung 100 µl der zu bestimmenden Probe gegeben und 1 Stunde bei Raumtemperatur unter Schütteln inkubiert.

Nach 3maligem Waschen mit 0.9% Natriumchlorid/0.05% Tween 20 werden je Vertiefung 100 µl des Antikörpers gegen glykiertes Albumin (1 bzw. 5 µg/ml) zugegeben und 1 Stunde bei Raumtemperatur unter Schütteln inkubiert. Anschließend wird erneut 3 mal gewaschen und dann gebundener Antikörper durch Zugabe von 100 µl je Vertiefung eines Peroxidase-markierten polyklonalen Antiserums aus dem Schaf gegen FCγ der Maus (immunsorbtiv gereinigt, 20 mU/ml) nachgewiesen. Hierzu wird 1 Stunde bei Raumtemperatur unter Schütteln inkubiert, anschließend 3 mal gewaschen und die Nachweisreaktion durch Zugabe von 100 µl je Vertiefung ABTS® ausgelöst. Nach 30 min bei Raumtemperatur erfolgt die Messung bei 405/490 nm im Dynatech-ELISA-Reader MR700. Die Menge an glykiertem Albumin in der zu analysierenden Probe kann dann durch Vergleich der Extinktion ermittelt werden, die bei Zugabe einer bekannten Menge an glykiertem Albumin erhalten wird.

### Beispiel 5

### Bestimmung der Epitopüberlappung von Antikörpern gegen glykiertes Albumin

Zum Nachweis der Epitopüberlappung eines Antikörpers mit dem monoklonalen Antikörper MAK 2.21.11 (DSM ACC 2052) wird ein kompetetiver Enzym-Immunoassay durchgeführt. Dazu wird das gemäß Beispiel 1 isolierte glykierte Albumin zunächst mit D-Biotinyl-ε-amidocapronsäure-N-hydroxysuccinimidester (Boehringer Mannheim, Katalog-Nr. 1008960) gemäß Angaben des Herstellers biotinyliert. Von diesem biotinylierten Antigen werden 300 ng in einem Volumen von 100 µl PBS durch einstündige Inkubation bei Raumtemperatur an eine mit Streptavidin beschichtete Mikrotiterplatte (Herstellung nach EP-A 0 344 578) gebunden. Nach viermaligem Waschen mit PBS/0,05 % Tween 20 wird 90 Minuten bei Raumtemperatur simultan inkubiert mit dem monoklonalen Antikörper MAK 2.21.11, der mit Peroxidase markiert wurde (Endkonzentration 250 mU/ml) und dem zu beurteilenden Antikörper. Nach erneutem viermaligem Waschen mit PBS/0,05 % Tween 20 wird mit der Enzymsubstratlösung ABTS® in Natriumperborat enthaltendem Puffer (Boehringer Mannheim GmbH, Katalog Nr. 687251 und 687359) 30 Minuten bei Raumtemperatur inkubiert und anschließend die Extinktion bei 405 nm als Maß für die Menge des gebundenen, POD markierten, monoklonalen Antikörpers MAK 2.21.11 gemessen. Dieser Wert wird verglichen mit der Extinktion, die erhalten wird, bei Inkubation mit dem monoklonalen Antikörper MAK 2.21.11 allein. Wenn bis zu einem 10⁵-fachen Überschuß an zu beurteilendem Antikörper gegenüber dem monoklonalen Antikörper MAK 2.21.11 Enzymkonjugat (250 mU/ml) mindestens 50 % Kompetition zu erkennen sind, liegt eine Epitopüberlappung vor.

## Patentansprüche

1. Monoklonaler Antikörper gegen in vivo glykiertes Albumin, dadurch gekennzeichnet, daß er zu weniger als 30 % mit nicht glykiertem Albumin reagiert und erhältlich ist durch Immunisierung mit in vivo glykiertem Albumin, das aus Serum über Immunaffinitätschromatographie und Boronatchromatographie aufgereinigt wurde, Immortalisierung der Milzzellen der immunisierten Tiere und Klonierung derjenigen immortalisierten Zellen, deren Kulturüberstand den gewünschten Antikörper gegen glykiertes Albumin enthält.

2. Monoklonaler Antikörper gemäß Anspruch 1, dadurch gekennzeichnet, daß er zu weniger als 25 % mit nicht glykiertem Albumin reagiert.

3. Monoklonaler Antikörper gemäß einem der Ansprüche 1 oder 2, dadurch gekennzeichnet, daß er zu weniger als 10 % mit nicht glykiertem Albumin reagiert.

4. Monoklonaler Antikörper gemäß einem der Ansprüche 1 bis 3, dadurch gekennzeichnet, daß er zu weniger als 1 % mit nicht glykiertem Albumin reagiert.

5. Monoklonaler Antikörper gemäß einem der Ansprüche 1 bis 4, dadurch gekennzeichnet, daß er in äquivalenter Weise an glykiertes Albumin bindefähig ist wie der von der Zellinie MAK 2.21.11 (DSM ACC 2052) oder MAK 2.6.28 (DSM ACC 2053) produzierte monoklonale Antikörper.

6. Monoklonaler Antikörper gemäß einem der Ansprüche 1 bis 5, erhältlich aus der Zellinie MAK 2.21.11 (DSM ACC 2052) oder MAK 2.6.28 (DSM ACC 2053).

7. Zellinie MAK 2.21.11 (DSM ACC 2052).

8. Zellinie MAK 2.6.28 (DSM ACC 2053).

9. Verfahren zur Herstellung eines monoklonalen Antikörpers gemäß einem der Ansprüche 1 bis 6, dadurch gekennzeichnet, daß man mit in vivo glykiertem Albumin immunisiert, das aus Serum über Immunaffinitätschromatographie und Boronatchromatographie aufgereinigt wurde, die Milzzellen der immunisierten Tiere immortalisiert und diejenigen immortalisierten Zellen kloniert, deren Kulturüberstand den gewünschten Antikörper gegen glykosyliertes Albumin enthält.

10. Verfahren zur Aufreinigung von in vivo glykiertem Albumin, dadurch gekennzeichnet, daß man Albumin aus Serum über Immunaffinitätschromatographie aufreinigt und das in vivo glykierte Albumin über eine Boronatchromatographie abtrennt.

11. Verwendung eines monoklonalen Antikörpers gemäß einem der Ansprüche 1 bis 6 in einem immunologischen Nachweisverfahren zur spezifischen Bestimmung von in vivo glykiertem Albumin.

12. Verwendung eines monoklonalen Antikörpers gemäß einem der Ansprüche 1 bis 6 zum spezifischen Nachweis von in vivo glykiertem Albumin durch Inkubation der zu analysierenden Probe mit dem Antikörper, Immobilisierung der gebildeten Komplexe vor oder nach der Bindungsreaktion und Bestimmung der Bindung des Antikörpers an das Antigen in üblicher Weise.

13. Immunologisches Verfahren zur spezifischen Bestimmung von in vivo glykiertem Albumin, dadurch gekennzeichnet, daß mindestens ein monoklonaler Antikörper gemäß einem der Ansprüche 1 bis 6 verwendet wird.

14. Immunologisches Verfahren gemäß Anspruch 13, dadurch gekennzeichnet, daß man die zu analysierende Probe mit dem Antikörper inkubiert, die gebildeten Komplexe vor oder nach der Bindungsreaktion immobilisiert und die Bindung des Antikörpers an das Antigen in üblicher Weise bestimmt.
